(19) Europäisches Patentamt
European Patent Office
Office européen des brevets

(11) EP 0 821 586 B1

(12) **EUROPEAN PATENT SPECIFICATION**

(45) Date of publication and mention of the grant of the patent:
**11.06.2003 Bulletin 2003/24**

(21) Application number: **96910803.4**

(22) Date of filing: **11.04.1996**

(51) Int Cl.[7]: **A61K 31/425**, A61K 31/415, A61P 35/00, A61P 35/02

(86) International application number:
**PCT/US96/04955**

(87) International publication number:
**WO 96/032107 (17.10.1996 Gazette 1996/46)**

(54) **A PHARMACEUTICAL COMPOSITION CONTAINING BENZIMIDAZOLE FOR INHIBITING THE GROWTH OF CANCERS**

BENZIMIDAZOLDERIVATEN-ENTHALTENDE PHARMAZEUTISCHE ZUSAMMENSETZUNGEN ZUR INHIBIERUNG DES KREBSWACHSTUMS

COMPOSITION PHARMACEUTIQUE CONTENANT DU BENZIMIDAZOLE POUR INHIBER LE DEVELOPPEMENT DES CANCERS

(84) Designated Contracting States:
**AT BE CH DE DK ES FI FR GB GR IE IT LI LU NL PT SE**

(30) Priority: **12.04.1995 US 420914**
**07.06.1995 US 473817**
**03.08.1995 US 1837**

(43) Date of publication of application:
**04.02.1998 Bulletin 1998/06**

(73) Proprietor: **The University of Arizona Foundation Tucson, Arizona 85721 (US)**

(72) Inventor: **CAMDEN, James, Berger West Chester, OH 45069 (US)**

(74) Representative: **Raynor, John et al**
**W.H. Beck, Greener & Co**
**7 Stone Buildings**
**Lincoln's Inn**
**London WC2A 3SZ (GB)**

(56) References cited:
**US-A- 5 114 951**

- **CANCER TREAT.REP., vol. 62, no. 11, 1978, pages 1955-62, XP000579564 LUNDY ET AL.: "Immunomodulation with thiabendazole: a review of immunologic properties and efficacy in combined modality cancer therapy"**
- **DISS.ABSTR.INT.(SCI.), vol. 39, no. 11, 1979, pages 5315-5316, XP000579587 LOVETT: "Immunomodulating effects of thiabendazole: immunotherapeutic efficacy in the treatment of a murine fibrosarcoma"**
- **SURG.FORUM, vol. 27, no. 62, 1976, pages 132-4, XP002011468 LUNDY ET AL.: "Thiabendazole: a new immunopotentiator effective in therapy of murine fibrosarcoma"**
- **TOXICOLOGY, vol. 94, no. 1-3, 1994, pages 173-85, XP000579566 MARINOVICH ET AL.: "Mixtures of benomyl, pirimphos-methyl, dimethoate, diazinon, and azinphos-methyl affect the protein synthesis in HL-60 cells differently"**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 821 586 B1

Printed by Jouve, 75001 PARIS (FR)

## Description

### TECHNICAL FIELD

This invention is the use of a pharmaceutical composition for the preparation of a medicament that inhibits the growth of cancers and tumors, including leukemia, in mammals, particularly in human and warm blooded animals. The composition contains a benzimidazole derivative.

### BACKGROUND OF THE INVENTION

Cancers are the leading cause of death in animals and humans. The exact cause of cancer is not known, but links between certain activities such as smoking or exposure to carcinogens and the incidence of certain types of cancers and tumors has been shown by a number of researchers.

Many types of chemotherapeutic agents have been shown to be effective against cancers and tumor cells, but not all types of cancers and tumors respond to these agents. Unfortunately, many of-these agents also destroy normal cells. The exact mechanism for the action of these chemotherapeutic agents are not always known.

Despite advances in the field of cancer treatment the leading therapies to date are surgery, radiation, chemotherapy and bone marrow transplants. Chemotherapeutic approaches are said to fight cancers that are metastasized or ones that are particularly aggressive. Such cytocidal or cytostatic agents work best on cancers with large growth factors, i.e., ones whose cells are rapidly dividing. To date, hormones, in particular estrogen, progesterone and testosterone, and some antibiotics produced by a variety of microbes, alkylating agents, and anti-metabolites form the bulk of therapies available to oncologists. Ideally cytotoxic agents that have specificity for cancer and tumor cells while not affecting normal cells would be extremely desirable. Unfortunately, none have been found and instead agents which target especially rapidly dividing cells (both tumor and normal) have been used.

Clearly, the development of materials that would target tumor cells due to some unique specificity for them would be a breakthrough. Alternatively, materials that were cytotoxic to tumor cells while exerting mild effects on normal cells would be desirable. Therefore, it is an object of this invention to provide a pharmaceutical composition that is effective in inhibiting the growth of tumors and cancers in mammals with mild or no effects on normal cells.

More specifically, it is an object of this invention to provide an anti-cancer composition for the manufacture of a medicament comprising a pharmaceutical carrier and a benzimidazole derivative as defined herein.

The development of materials that would target leukemia cells due to some unique specificity for them would also be a breakthrough. Alternatively, materials that were cytotoxic to leukemia cells while exerting mild effects on normal cells would be desirable. Therefore, it is an object of this invention to provide a pharmaceutical composition that is effective in treating leukemia with mild or no effects on normal blood cells.

More specifically, it is an object of this invention to provide a composition for the manufacture of a medicament comprising a pharmaceutical carrier and a benzimidazole derivative as defined herein for treating leukemia.

It is believed that these benzimidazole compositions when used in conjunction with chemotherapeutic agents can reduce the growth of cancers and tumors, including leukemia. It has been found that the benzimidazoles are especially effective in suppressing the growth of the cancer, tumor, or bacteria. The use of these benzimidazoles in combination with other chemotherapeutic agents which are effective in destroying the tumor is a novel method of treatment.

More specifically, it is an object of this invention to provide an anti-cancer composition for the manufacture of a medicament comprising a pharmaceutical carrier and a benzimidazole derivative and a chemotherapeutic agent as defined herein. A potentiator can be used in these compositions.

These and other objects will become evident from the following detailed description of this invention.

### SUMMARY OF THE INVENTION

The use of a pharmaceutical composition for the manufacture of a medicament for treatment of cancers, tumors, including leukemia in mammals, and in particular, warm blooded animals and humans, comprising a pharmaceutical carrier and an effective amount of an anti-cancer compound selected from the group consisting of:

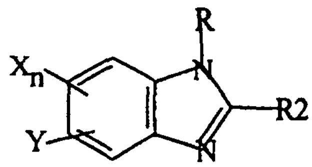

wherein X is hydrogen, halogen, alkyl of less than 7 carbon atoms, or alkoxy of less than 7 carbon atoms; n is a positive integer of less than 4; Y is hydrogen, chloro, nitro, methyl or ethyl; R is hydrogen, or an alkyl group of from 1 to 8 carbon atoms; and $R_2$ is $NHCOOR_1$ wherein $R_1$ is aliphatic hydrocarbon of less than 7 carbon atoms, and preferably an alkyl group of less than 7 carbon atoms is claimed.

These compositions can be used to inhibit the growth of cancers and other tumors in humans or animals by administration of an effective amount either orally, rectally, topically, parenterally, intravenously, or by injection into the tumor. These compositions do not significantly affect healthy cells as compared to adriamycin, which has a detrimental effect on healthy cells.

The use for the preparation of a medicament of a pharmaceutical composition for treatment of mammals, and in particular warm blooded animals and humans, comprising a pharmaceutical carrier and an effective amount of chemotherapeutic agents and a benzimidazole as described above is also encompassed.

Potentiators can also be used with this composition.

Preferably, the compositions are:

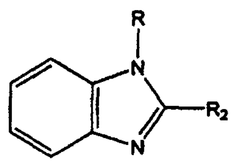

wherein R is an alkyl of 1 through 8 carbon atoms, and $R_2$ is $NHCOOR_1$ wherein $R_1$ is methyl, ethyl, or isopropyl; or the non-toxic, pharmaceutically acceptable acid addition salts thereof with an organic or inorganic acid. The most preferred compound is 2-methoxycarbonylaminobenzimidazole, and those wherein Y is chloro.

## DETAILED DESCRIPTION OF THE INVENTION

### A. DEFINITIONS:

As used herein, a "pharmaceutically acceptable" component is one that is suitable for use with humans and/or animals without undue adverse side effects (such as toxicity, irritation, and allergic response) commensurate with a reasonable benefit/risk ratio.

As use herein, the term "safe and effective amount" refers to the quantity of a component which is sufficient to yield a desired therapeutic response without undue adverse side effects (such as toxicity, irritation or allergic response) commensurate with a reasonable benefit/risk ratio when used in the manner of this invention. The specific "safe and effective amount" will, obviously, vary with such factors as the particular condition being treated, the physical condition of the patient, the type of mammal being treated, the duration of the treatment, the nature of concurrent therapy (if any), and the specific formulations employed and the structure of the compounds or its derivatives.

As used herein, a "pharmaceutical addition salts" is salt of the anti-cancer compound with an organic or inorganic acid. These preferred acid addition salts are chlorides, bromides, sulfates, nitrates, phosphates, sulfonates, formates, tartrates, maleates, malates, citrates, benzoates, salicylates, ascorbates, or the like.

As used herein, a "pharmaceutical carrier" is a pharmaceutically acceptable solvent, suspending agent or vehicle, including liposomes, for delivering the anti-cancer agent to the animal or human. The carrier may be liquid or solid and is selected with the planned manner of administration in mind.

As used herein, "cancer" refers to all types of cancers or neoplasm or malignant tumors found in mammals, including tumors and leukemia. Cancers include those malignant diseases which attack normal healthy cells. Leukemia encompasses those diseases which attack normal healthy blood cells and bone marrow which produce bone cells which are found in mammals.

As used herein, the "anti-cancer compounds" are the benzimidazoles, and their salts. The exact benzimidazoles are described in detail below. The preferred material is the product sold under the name "carbendazim®" by

BASF or Hoechst, DuPont or MSD-AgVet.

As used herein "chemotherapeutic agents includes DNA-interactive Agents, Antimetabolites, Tubulin-Interactive Agents, Hormonal agents and others, such as Asparaginase or hydroxyurea.

As used herein "potentiators" are materials such as triprolidine and its cis-isomer and procodazole which are used in combination with the chemotherapeutic agents and benzimidazoles.

**B. THE ANTI-CANCER COMPOUNDS**

The anti-cancer compounds are benzimidazole derivatives which are known for their antifungal activities. They are systemic fungicides used to prevent and eradicate fungi. The compounds have the following structure:

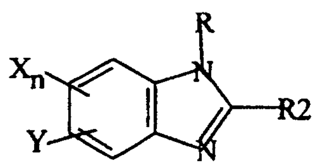

wherein X is hydrogen, halogen, alkyl of less than 7 carbon atoms or alkoxy of less than 7 carbon atoms; n is a positive integer of less than 4; Y is hydrogen, chloro, nitro, methyl or ethyl; and R is hydrogen, or an alkyl group of from 1 to 8 carbon atoms; and $R_2$ is $NHCOOR_1$ wherein $R_1$ is aliphatic hydrocarbon or less than 7 carbon atoms, and preferably an alkyl group of less than 7 carbon atoms.

Preferably, the compositions are:

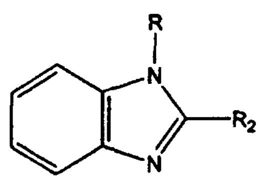

wherein R is an alkyl of 1 through 8 carbon atoms, and $R_2$ is $NHCOOR_1$ wherein $R_1$ is methyl, ethyl, or isopropyl; or the non-toxic, pharmaceutically acceptable acid addition salts thereof with an organic or inorganic acid.

The most preferred compound is 2-methoxycarbonylaminobenzimidazole, and the compounds wherein Y is chloro and X is hydrogen.

These compounds are prepared according to the method described in U.S. 3,738,995 issued to Adams et al., June 12, 1973.

It is believed that fungicides, particularly systemic fungicides, have the capability of reducing tumors or decreasing their growth significantly. Systemic fungicides have the ability to migrate through the plant or animal body. While this is a positive attribute, it is not an essential requirement of the effective compounds for treating cancers, or tumors.

**C. CHEMOTHERAPEUTIC AGENTS**

The chemotherapeutic agents are generally grouped as DNA-interactive Agents, antimetabolites, tubulin-interactive agents, hormonal agents and others such as asparaginase or hydroxyurea. Each of the groups of chemotherapeutic agents can be further divided by type of activity or compound. The chemotherapeutic. agents used in combination with the anti-cancer agents or benzimidazoles of this invention include members of all of these groups. For a detailed discussion of the chemotherapeutic agents and their method of administration, see Dorr, et al., *Cancer Chemotherapy Handbook*, 2d edition, pages 15-34, Appleton & Lange (Connecticut, 1994) herein incorporated by reference.

DNA-Interactive Agents include the alkylating agents, e.g., Cisplatin, Cyclophosphamide, Altretamine; the DNA strand-breakage agents, such as Bleomycin; the intercalating topoisomerase II inhibitors, e.g., Dactinomycin and Doxorubicin); the nonintercalating topoisomerase II inhibitors, such as Etoposide and Teniposide; and the DNA minor groove binder Plcamycin.

The alkylating agents form covalent chemical adducts with cellular DNA, RNA. and protein molecules and

with smaller amino acids, glutathione and similar chemicals. Generally, these alkylating agents react with a nucleophilic atom in a cellular constituent, such as an amino, carboxyl, phosphate, sulfhydryl group in nucleic acids, proteins, amino acids, or glutathione. The mechanism and the role of these alkylating agents in cancer therapy is not well understood. Typical alkylating agents include:

Nitrogen mustards, such as Chlorambucil, Cyclophosphamide, Isofamide, Mechlorethamine, Melphalan, Uracil mustard;
aziridines such as Thiotepa;
methanesulfonate esters such as Busulfan;
nitroso ureas, such as Carmustine, Lomustine, Streptozocin;
platinum complexes, such as Cisplatin, Carboplatin;
bioreductive alkylator, such as Mitomycin, and Procarbazine, Dacarbazine and Altretamine;
DNA strand breaking agents include Bleomycin;
DNA topoisomerase II inhibitors include the following:

Intercalators, such as Amsacrine, Dactinomycin, Daunorubicin, Doxorubicin, Idarubicin, and Mitoxantrone;
nonintercalators, such as Etoposide and Teniposide.

The DNA minor groove binder is Plicamycin.

The antimetabolites interfere with the production of nucleic acids by one or the other of two major mechanisms. Some of the drugs inhibit production of the deoxyribonucleoside triphosphates that are the immediate precursors for DNA synthesis, thus inhibiting DNA replication. Some of the compounds are sufficiently like purines or pyrimidines to be able to substitute for them in the anabolic nucleotide pathways. These analogs can then be substituted into the DNA and RNA instead of their normal counterparts. The antimetabolites useful herein include:

folate antagonists such as Methotrexate and trimetrexate
pyrimidine antagonists, such as Fluorouracil, Fluorodeoxyuridine, CB3717, Azacytidine, Cytarabine, and Floxuridine
purine antagonists include Mercaptopurine, 6-Thioguanine, Fludarabine, Pentostatin;
sugar modified analogs include Cyctrabine, Fludarabine;
ribonucleotide reductase inhibitors include hydroxyurea.

Tubulin Interactive agents act by binding to specific sites on tubulin, a protein that polymerizes to foam cellular microtubules. Microtubules are critial cell structure units. When the interactive agents bind on the protein, the cell can not form microtubules Tubulin Interactive agents include Vincristine and Vinblastine, both alkaloids and Paclitaxel.

Hormonal agents are also useful in the treatments of cancers and tumors. They are used in hormonally susceptible tumors and are usually derived from natural sources. These include:

estrogens, conjugated estrogens and Ethinyl Estradiol and Diethylstilbesterol, Chlorotrianisene and Idenestrol;
progestins such as Hydroxyprogesterone caproate, Medroxyprogesterone, and Megestrol;
adrogens such as testosterone, testosterone propionate; fluoxymesterone, methyltestosterone;

Adrenal corticosteroids are derived from natural adrenal cortisol or hydrocortisone. They are used because of their anti inflammatory benefits as well as the ability of some to inhibit mitotic divisions and to halt DNA synthesis. These compounds include, Prednisone, Dexamethasone, Methyleprednisolone, and Prednisolone.

Leutinizing hormone releasing hormone agents or gonadotropin-releasing hormone antagonists are used primarily the treatment of prostate-cancer. These include leuprolide acetate and goserelin acetate. They prevent the biosynthesis of steroids in the testes.

Antihormonal agents include:

antiestrogenic agents such as Tamosifen,
antiandrogen agents such as Flutamide; and
antiadrenal agents such as Mitotane and Aminoglutethimide.

Hydroxyurea appears to act primarily through inhibition of the enzyme ribonucleotide reductase.

Asparagenase is an enzyme which converts asparagine to nonfunctional aspartic acid and thus blocks protein synthesis in the tumor.

### D. POTENTIATORS

The "potentiators" can be any material which improves or increase the efficacy of the pharmaceutical composition and/or an immuno suppressor. One such potentiator is triprolidine and its cis-isomer which are used in combination with the chemotherapeutic agents and the benzimidazole. Triprolidine is described in US 5,114,951 (1992). Another potentiator is procodazole, 1H-Benzimidazole-2-propanoic acid; [β-(2-benizidazole) propionic acid; 2-(2-carboxyethyl) benzimidazole; propazol) Procodazole is a non-specific active immunoprotective agent bacterial infections and can be used with the compositions claimed herein. It is effective with the benzimidazoles alone in treating cancers, tumors, or leukemia or with the combined benzimidazoles and chemotherapeutic agents. Propionic acid and its salts and esters can also be used in combination with the pharmaceutical compositions claimed herein.

The potentiators also improve the efficacy of the benzimidazole compounds in conjunction with these anticancers agents in a

safe and effective amount. These combinations can be administered to the patient or animal by oral, rectal, topical or parenteral administration.

Antioxidant vitamins such as ascorbic acid, beta-carotene, vitamin A and vitamin E can be administered with the compositions of this invention.

### E. DOSAGE

Any suitable dosage may be given in the invention. The type of compounds and the carriers and the amount will vary widely depending on the species of the warm blooded animal or human, body weight, and cancer, or tumor being treated. The range and ratio of the chemotherapeutic agent and anti cancer compound used will depend on the type of chemotherapeutic agent and the cancer being treated. Generally a dosage of between about 2 milligrams (mg) per kilogram (kg) of body weight and about 400 mg per kg of body weight is suitable. Preferably from 15 mg to about 150 mg/kg of body weight is used for the benzimidazoles. For the chemotherapeutic agents, a lower dosage may be appropriate, i.e., from about 0.5 mg/kg of body weight to about 400 mg/kg body weight. Generally, the dosage in man is lower than for small warm blooded mammals such as mice. A dosage unit may comprise a single compound or mixtures thereof with other compounds or other cancer inhibiting compounds. The dosage unit can also comprise diluents, extenders, carriers and the like. The unit may be in solid or gel form such as pills, tablets, capsules, liposomes and the like or in liquid form suitable for oral, rectal, topical, intravenous injection or parenteral administration or injection into or around the tumor or into or around the bone marrow.

### F. DOSAGE DELIVERY FORMS

The anti-cancer compounds and chemotherapeutic agents are typically mixed with a pharmaceutically acceptable carrier. This carrier can be a solid or liquid or a liposome and the type is generally chosen based on the type of administration being used. The active agent can be coadministered in the form of a tablet or capsule, liposome, or as an agglomerated powder or in a liquid form. Examples of suitable solid carriers include lactose, sucrose, gelatin and agar. Capsule or tablets can be easily formulated and can be made easy to swallow or chew; other solid forms include granules, and bulk powders. Tablets may contain suitable binders, lubricants, diluents, disintegrating agents, coloring agents, flavoring agents, flow-inducing agents, and melting agents. Examples of suitable liquid dosage forms include solutions or suspensions in water, pharmaceutically acceptable fats and oils, alcohols or other organic solvents, including esters, emulsions, syrups or elixirs, suspensions, solutions and/or suspensions reconstituted from non-effervescent granules and effervescent preparations reconstituted from effervescent granules. Such liquid dosage forms may contain, for example, suitable solvents, preservatives, emulsifying agents, suspending agents, diluents, sweeteners, thickeners, and melting agents. Oral dosage forms optionally contain flavorants and coloring agents. Parenteral and intravenous forms would also include minerals and other materials to make them compatible with the type of injection or delivery system chosen.

Specific examples of pharmaceutical acceptable carriers and excipients that may be used to formulate oral dosage forms of the present invention are described in US. Pat No. 3,903,297 to Robert, issued Sept. 2, 1975. Techniques and compositions for making dosage form useful in the present invention are described in the following references: 7 Modern Pharmaceutics, Chapter 9 and 10 (Banker & Rhodes, Editors, 1979); Lieberman et al., Pharmaceutical Dosage Forms: Tablets (1981); and Ansel, Introduction to Pharmaceutical Dosage Forms $2^{nd}$ Edition (1976).

### G METHOD OF TREATMENT

The method of treatment can be any suitable method which is effective in the treatment of the particular cancer or tumor that is being treated. Treatment may be oral, rectal, topical, parenteral or intravenous administration or by

injection into the tumor and the like. The method of applying an effective amount also varies depending on the tumor being treated. It is believed that parental treatment by intravenous, subcutaneous, or intramuscular application of the benzimodale compounds, formulated with an appropriate carrier, additional cancer inhibiting compound or compounds or diluent to facilitate application will be the preferred method of administering the compounds to warm blooded animals.

These same systemic fungicides can be used alone or in combination with other fungicides along with the chemotherapeutic agents.

Other fungicides that can be used with these materials include 1h-1, 2, 4-triazole derivatives such as fluconazole, and propiconazole, and N-chlorophenythiocarbamates. Herbicides such as N-phosphonoglycines derivative, e.g. glyphoste can also be used in combination with the benzimidazoles.

The following examples are illustrated and are not meant to be limiting to the invention.

Example I

Colon, Breast and Lung Tumor Cells Test

The following cell culture tests were performed to test the toxicity of the benzimidazole compounds on colon, breast and lung human tumor cells. The viability of the cells were tested by looking at MTT (3-[4,5-dimenthylthiazol-2-yl] -2,5-diphenyltetrazolium bromide) reduction. MTT assay is a well known measure of cell viability.

The colon tumor cells (HT29 from American Tyle Culture Collection (ATCC) and the breast cells (MX1 from cells lines from ATCC) were cultured in Eagle's Miminal Essential Medium with 10% fetal bovine serum. The lung tumor cells (A549 from ATCC cell lines) were cultured in Ham's F12 medium with 10% fetal bovine serum.

The tumor cells were passaged and seeded into culture flasks at the desired cell densities. The culture medium was decanted and the cell sheets were washed twice with phosphate buffered saline (PBS). The cells were trypsinized and triturated prior to seeding the flasks. Unless otherwise indicated the cultures were incubated at 37° ± 1°C in a humidified atmosphere of 5± 1% carbon dioxide in air. The cultures were incubated until they were 50-80% confluent.

The cells were subcultured when the flasks were subconfluent. The medium was aspirated from the flasks and the cell sheets rinsed twice with PBS. Next, the Trypsin Solution was added to each flask to cover the cell sheet. The Trypsin Solution was removed after 30-60 seconds and the flasks were incubated at room temperature for two to six minutes. When 90% of the cells became dislodged, growth medium was added. The cells were removed by trituration and transferred to a sterile centrifuge tube. The concentration of cells in the suspension was determined, and an appropriate dilution was made to obtain a density of 5000 cells/ml. The cells were subcultured into the designated wells of the 96-well bioassay plates (200 microliter cell suspension per well). PBS was added to all the remaining wells to maintain humidity. The plates were then incubated overnight before test article treatment.

Each dose of test article was tested by treating quadruplicate wells of cultures with 100 microliter of each dilution. Those wells designated as solvent controls received an additional 100 microliter of methanol control; negative controls wells received an additional 100 microliters of treatment medium. PBS was added to the remaining wells not treated with test article or medium. The plates were then incubated for approximately 5 days.

At the end of the 5 day incubation, each dose group was examined microscopically to assess toxicity. A 0.5 mg/ml dilution of MTT was made in treatment medium, and the dilution was filtered through a 0.45 micrometer filter to remove undissolved crystals. The medium was decanted from the wells of the bioassy plates. Immediately thereafter, 2000 microliter of the filtered MTT solution was added to all test wells except for the two untreated blank test wells. The two blank wells received 200 microliters of treatment medium. The plates were returned to the incubator for about 3 hours. After incubation, the MTT containing medium was decanted. Excess medium was added to each well and the plates were shaken at room temperature for about 2 hours.

The absorbance at 550 nm ($OD_{550}$) of each well was measured with a Molecular Devices (Menlo Park, CA) VMax plate reader.

The mean $OD_{550}$ of the solvent control wells and that of each test article dilution, and that of each of the blank wells and the positive control were calulated. The mean $OD_{550}$ of the blank wells were subtracted from the mean of the solvent contrl wells, and test srticle wells, respectively to give teh corresponding mean $OD_{550}$.

$$\text{\% of Control} = \frac{\text{corrected mean } OD_{550} \text{ of Test Article Dilution}}{\text{corrected mean of } OD_{550} \text{ of Solvent Control}} \times 100$$

Dose response curves were prepared as semi-log plots with % of control on the ordinate (linear) and the test article concentration on the abscissa (logarithmic). The $EC_{50}$ was interpolated from the plots for each test article.

For the test articles administered in methanol, separate were prepared to correct for the methanol data.

Adriamycin was used as a positive control. In all cases, it was more toxic than any of the test materials by

one or two logs. Adriamycin is one of the more potent agents in current use and one with significant side effects. The peak plasma concentration of other, quite effective chemotherapeutic agents may be 10 to 50 times higher than that of Adriamycin.

The $EC_{50}$ is the concentration at which one half of the calls are killed.

Table 1

| Test Material | EC-50 Resuli (ppm) | | | | | |
|---|---|---|---|---|---|---|
| | HT29 | HT29 | MX1 | MX1 | A549 | A549 |
| Benomyl | 0.728 | 0.682 | 3.26 | 2.4 | 3.24 | 2.81 |
| Carbendazin | 0.320 | 0.506 | 0.752 | 0.822 | 1.52 | 1.42 |
| Adriamycin | 0.015 | 0.0020 | 0.0035 | 0.0093 | 0.065 | 0.10 |

In normal healthy cells, the following results were obtained. As is evident, the benomyl and carbendazim were much less toxic to normal heathly cells than adriamycin.

**Table 2**

| **Test Material** | **EC-50** | | | | | |
|---|---|---|---|---|---|---|
| | **Broncheal Cells** | | **Kerotinoyle Cells** | | **Fibroblasts** | |
| **Benomyl** | **0.728** | **0.682** | **3.26** | **2.4** | **3.24** | **2.81** |
| **Carbendazin** | **0.320** | **0.506** | **0.752** | **0.822** | **1.52** | **1.42** |
| **Adriamycin** | **0.015** | **0.0020** | **0.0035** | **0.0093** | **0.065** | **0.10** |

Example II

Mice are randomly selected and divided into groups for treatment. Five groups are infected with leukemia. The diseased animals are dosed for five days, off two days, then dosed for another five days and then three days off, then dosed for five days and off for two days. This dosing on and off in an irregular pattern was not an ideal regimen, but the results do show a positive benefit for the Carbendazim™. One group of mice was treated with Cytoxan™, 2-[bis (2-chloroethyl)amino-1-oxo-2-aza-5-oxophosphoridin, a control was dosed with canola oil, and three groups were treated with various levels of Carbendazim™, 2-methoxycarbonylaminobenzimidazole. A control with no treatment was also used. The Carbendazim was dosed at three levels: 4000 mg/kg, 2500 mg/kg, and 1000 mg/kg. The Cytoxan was dosed at 125 mg/kg. After 8 days, the no-treatment group had lost 1 mouse, by day 10 eight mice were dead, and at day 11 all ten mice were dead. The mice in the Cytoxan group survived more than 21 days. The higher dose Carbendazim group had one mouse die on day 14, two died on days 15, 16 and 17, and one each died on days 20, 21, and 22. The mean number of days for this group is 17.3. The intermediate dosage Carbendazim group had 2 mice die on day 14, 4 on day 15, 1 on day 16, 2 on day 19, and 1 on day 21. The mean number of days for this group is 16.50. The lowest Carbendazim dosage group had 2 mice die on day 12, 13, 14, and 15; and 1 died on each of days 16 and 17. The mean number of days for this group is 14.1.

**Claims**

**1.** Use of a benzimidazole derivative of the formula:

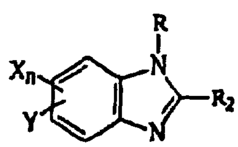

wherein, X is hydrogen, halogen, alkyl of less than 7 carbon atoms, or alkoxy of less than 7 carbon atoms;

n is a positive integer of less than 4; Y is hydrogen, chloro, nitro, methyl, or ethyl; R is hydrogen or an alkyl group having from 1 to 8 carbon atoms; and $R_3$ is $NHCOOR_1$ wherein $R_1$ is an aliphatic hydrocarbon of less than 7 carbon atoms;

or a pharmaceutical addition salt thereof,

for the manufacture of a medicament for the inhibition or treatment of a cancer of a tumor.

**2.** The use according to Claim 1 wherein $R_1$ is methyl, ethyl or isopropyl.

**3.** The use according to Claim 1 wherein said benzimidazole derivative is 2-methoxycarbonylaminobenzimidazole or a pharmaceutical addition salt thereof.

**4.** The use according to Claim 1, or 2 wherein said pharmaceutical addition salt is selected from the group consisting of chlorides, bromides, sulfates, nitrates, phosphates, sulfonates, formates, tartrates, maleates, malates, citrates, benzoates, salicylcates, ascorbates, and a mixture thereof. and wherein Y is chloro.

**5.** The use according to Claim 1, 2, or 3 wherein said benzimidazole derivative is present in said medicament in an amount to provide from 2 mg/kg body weight to 4000 mg/kg body weight.

**6.** The use according to Claim 1, 2, or 3 wherein said benzimidazole derivative is prepared for oral, enteric, intravenous, or parenteral administration, or for injection.

**7.** The use according to any of Claims 1 to 6 wherein the tumor is a malignant tumor.

**8.** The use according to any of Claims 1 to 7 wherein the medicament is for inhibiting the growth of a tumor.

**9.** The use according to any of Claims 1 to 8 wherein said cancer is leukemia.

**10.** The use according to any of Claims 1 to 8 wherein said cancer is colon cancer.

**11.** The use according to any of Claims 1 to 8 wherein said cancer is breast cancer.

**12.** The use according to any of Claims 1 to 8 wherein said cancer is lung cancer.

## Patentansprüche

**1.** Verwendung eines Benzimidazolderivats der Formel:

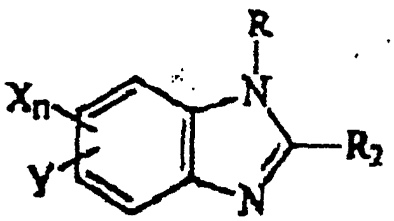

in welcher X für Wasserstoff, Halogen, Alkyl mit weniger als 7 Kohlenstoffatomen oder Alkoxy mit weniger als 7 Kohlenstoffatomen steht; n für eine positive ganze Zahl von weniger als 4 steht; Y für Wasserstoff, Chlor, Nitro, Methyl oder Ethyl steht; R für Wasserstoff oder eine Alkylgruppe mit 1 bis 8 Kohlenstoffatomen steht; und $R_2$ für $NHCOOR_1$ steht, wobei $R_1$ für einen aliphatischen Kohlenwasserstoff mit weniger als 7 Kohlenstoffatomen steht; oder eines pharmazeutischen Additionssalzes davon;
zur Herstellung eines Medikaments zur Inhibierung bzw. Behandlung einer Krebserkrankung bzw. eines Tumors.

**2.** Verwendung nach Anspruch 1, wobei $R_1$ für Methyl, Ethyl oder Isopropyl steht.

**3.** Verwendung nach Anspruch 1, wobei es sich bei dem Benzimidazolderivat um 2-Methoxycarbonylaminobenzimidazol oder ein pharmazeutisches Additionssalz davon handelt.

4. Verwendung nach Anspruch 1 oder 2, wobei das pharmazeutische Additionssalz aus der aus Chloriden, Bromiden, Sulfaten, Nitraten, Phosphaten, Sulfonaten, Formiaten, Tartraten, Maleaten, Malaten, Citraten, Benzoaten, Salicylaten, Ascorbaten und einer Mischung davon bestehenden Gruppe ausgewählt ist und wobei Y für Chlor steht.

5. Verwendung nach Anspruch 1, 2 oder 3, wobei das Benzimidazolderivat in dem Medikament in einer solchen Menge vorliegt, daß von 2 mg/kg Körpergewicht bis 4000 mg/kg Körpergewicht bereitgestellt werden.

6. Verwendung nach Anspruch 1, 2 oder 3, wobei das Benzimidazolderivat für eine orale, enterale, intravenöse oder parenterale Verabreichung oder für eine Injektion formuliert ist.

7. Verwendung nach einem der Ansprüche 1 bis 6, wobei es sich bei dem Tumor um einen malignen Tumor handelt.

8. Verwendung nach einem der Ansprüche 1 bis 7, wobei das Medikament zur Inhibierung des Wachstums eines Tumors bestimmt ist.

9. Verwendung nach einem der Ansprüche 1 bis 8, wobei es sich bei der Krebserkrankung um Leukämie handelt.

10. Verwendung nach einem der Ansprüche 1 bis 8, wobei es sich bei der Krebserkrankung um ein Kolonkarzinom handelt.

11. Verwendung nach einem der Ansprüche 1 bis 8, wobei es sich bei der Krebserkrankung um Brustkrebs handelt.

12. Verwendung nach einem der Ansprüche 1 bis 8, wobei es sich bei der Krebserkrankung um Lungenkrebs handelt.

**Revendications**

1. Emploi d'un dérivé de benzimidazole, de formule :

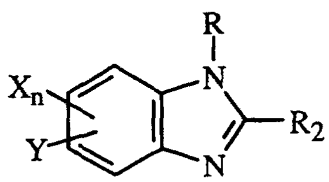

dans laquelle

X représente un atome d'hydrogène, un atome d'halogène, un groupe alkyle comportant moins de 7 atomes de carbone, ou un groupe alcoxy comportant moins de 7 atomes de carbone ;

n représente un nombre entier positif inférieur à 4 ;

Y représente un atome d'hydrogène ou de chlore ou un groupe nitro, méthyle ou éthyle ;

R représente un atome d'hydrogène ou un groupe alkyle comportant de 1 à 8 atomes de carbone ;

et $R_2$ représente un groupe de formule $-NHCOOR_1$ où $R_1$ représente un groupe hydrocarboné aliphatique comportant moins de 7 atomes de carbone ;

ou d'un sel d'addition d'un tel dérivé, utilisable en pharmacie, dans la fabrication d'un médicament destiné à inhiber ou à traiter un cancer ou une tumeur.

2. Emploi conforme à la revendication 1, $R_1$ représentant un groupe méthyle, éthyle ou isopropyle.

3. Emploi conforme à la revendication 1, ledit dérivé de benzimidazole étant du 2-méthoxycarbonylaminobenzimidazole ou un sel d'addition de celui-ci, utilisable en pharmacie.

4. Emploi conforme à la revendication 1 ou 2, ledit sel d'addition utilisable en pharmacie étant choisi parmi les chlorures, bromures, sulfates, nitrates, phosphates, sulfonates, formiates, tartrates, maléates, malates, citrates, benzoates, salicylates et ascorbates, ainsi que leurs mélanges, et Y représentant un atome de chlore.

**5.** Emploi conforme à la revendication 1, 2 ou 3, ledit dérivé de benzimidazole se trouvant présent dans ledit médicament en une quantité appropriée pour en fournir de 2 mg à 4 000 mg par kilogramme de poids corporel.

**6.** Emploi conforme à la revendication 1, 2 ou 3, ledit dérivé de benzimidazole étant formulé pour administration par voie orale, entérique, intraveineuse ou parentérale, ou pour injection.

**7.** Emploi conforme à l'une des revendications 1 à 6, la tumeur étant une tumeur maligne.

**8.** Emploi conforme à l'une des revendications 1 à 7, le médicament étant destiné à inhiber la croissance d'une tumeur.

**9.** Emploi conforme à l'une des revendications 1 à 8, ledit cancer étant une leucémie.

**10.** Emploi conforme à l'une des revendications 1 à 8, ledit cancer étant un cancer du côlon.

**11.** Emploi conforme à l'une des revendications 1 à 8, ledit cancer étant un cancer du sein.

**12.** Emploi conforme à l'une des revendications 1 à 8, ledit cancer étant un cancer du poumon.